# EUROPÄISCHE PATENTSCHRIFT

(11) **EP 2 465 844 B1**
(45) Veröffentlichungstag und Bekanntmachung des Hinweises auf die Patenterteilung: **09.07.2014**
(21) Anmeldenummer: 11193246.3
(22) Anmeldetag: 13.12.2011
(51) Int. Cl.: C07C 211/27, C07C 213/02, C07C 213/10, C07C 215/08, C07C 235/08, C07C 309/15, C07D 401/12, C07D 401/14, C07F 9/6584

(54) **Verfahren zur Herstellung und Aufreinigung von 3-Aminopropanol**
Method for producing and purifying 3-aminopropanol
Procédé de fabrication et de nettoyage de 3-aminopropanol

(30) Priorität: 14.12.2010 US 422673 P; 14.12.2010 EP 10194921
(43) Veröffentlichungstag der Anmeldung: 20.06.2012
(73) Patentinhaber: BASF SE, 67056 Ludwigshafen (DE)
(72) Erfinder: Kroll, Manfred, 67061 Ludwigshafen (DE); Herrmann, Andreas Edgar, 67346 Speyer (DE); Herbrecht, Dominik, 69124 Heidelberg (DE)

(56) Entgegenhaltungen:
- EP-A2- 1 120 404
- DRUGS.COM: 'Acamprosate', [Online] Gefunden im Internet: <URL:http://www.drugs.com/international/aca mprosate.html>

## Beschreibung

Die vorliegende Erfindung betrifft ein Verfahren zur Aufreinigung eines 3-Aminopropanol enthaltenden Reaktionsaustrags, der bei der Umsetzung von Ethylencyanhydrin mit Wasserstoff in Gegenwart von Ammoniak anfällt. Weiterhin betrifft die Erfindung die Herstellung von 3-Aminopropanol und dessen Verwendung. Ein weiterer Gegenstand der vorliegenden Erfindung ist ein Verfahren zur Herstellung von 3-Aminopropanolderivaten, insbesondere Panthenol, das dadurch gekennzeichnet ist, dass bei der Herstellung der 3-Aminopropanolderivate ein 3-Aminopropanol eingesetzt wird, das erfindungsgemäß aufgereinigt wurde.

3-Aminopropanol wird üblicherweise durch Umsetzung von Ethylencyanhydrin mit Wasserstoff hergestellt.

Die deutsche Patentschrift mit der Nr. 573983 offenbart die Hydrierung von Ethylencyanhydrin in Gegenwart von Hydrierkatalysatoren der Gruppen 8, 9 und 10 des periodischen Systems der Elemente. Nach der Hydrierung wird das Reaktionsprodukt vom Katalysator abgetrennt und durch fraktionierende Destillation aufgereinigt.

CH-B-244837 beschreibt die katalytische Reduktion von Nitrilen, u.a. Ethylencyanhydrin, welche in flüssigem Ammoniak gelöst oder suspendiert und dann unter Druck katalytisch hydriert wurden. Durch die Verwendung von flüssigem Ammoniak wird offenbarungsgemäß die Bildung von sekundären Basen zurückgedrängt, so dass als Hauptprodukt bei der Hydrierung primäres Amin entsteht. Nach Beendigung der Hydrierung wird Ammoniak abdestilliert, das Reaktionsprodukt vom Katalysator getrennt und anschließend im Vakuum destilliert.

In der DE-OS-2655794 wird ein weiteres Verfahren zur Herstellung von 3-Aminopropanol offenbart. In einer bevorzugten Ausführungsform des Verfahrens wird im Anschluss an die Ethylencyanhydrin-Synthese das erhaltene Produkt aminierend reduziert. Ammoniak wird in einem Überschuss von 10 bis 30 Mol je Mol Ethylencyanhydrin verwendet. Die Reduktion wird mit Wasserstoff in Gegenwart eines Hydrierkatalysators durchgeführt. Nach dem Ende der Reaktion wird das Reaktionsgemisch abgekühlt und gegebenenfalls filtriert. Aus dem Filtrat wird Aminopropanol durch Destillation im Vakuum abgetrennt.

In der europäischen Patentanmeldung EP-A1-1132371 wird ein Verfahren zur Herstellung von Alkanolaminen, u.a. vom 3-Aminopropanol, mit verbesserter Farbqualität beschrieben, in dem man die Alkanolamine in Gegenwart einer Phosphorverbindung bei verminderten Druck destilliert oder rektifiziert.

Ein weiteres Verfahren zur katalytischen Hydrierung von Ethylencyanhydrin wird in JP-A-2002201164 aufgeführt. Die Hydrierung wird in Gegenwart eines Raney-Cobalt-Katalysors und Ammoniak durchgefürht, wodurch die Bildung von sekundären und tertiären Aminen unterdrückt wurde, so dass ein reines Aminopropanol durch einfache Destillation erhalten werden konnte.

JP-A-2002053535 beschreibt die Destillation von Aminopropanol in Gegenwart von Tetrahydroboraten, um hochreines Aminopropanol mit geringen Anteilen an Morpholinen und Morpholinderivaten zu erhalten.

Die EP 1 120 404 beschreibt die Hydrierung von Hydroxypropionitril zu 3-Aminopropanol, welches geeignet ist für die Herstellung von Panthenol.

Die japanische Patentanmeldung JP-A-05163213 offenbart hingegen die Verwendung von Raney-Cobalt-Katalysatoren, um 3-Aminopropanol mit verbesserter Ausbeute zu erzielen.

3-Aminopropanol ist ein wichtiges Ausgangsprodukt für die Herstellung von Kosmetika, Pharmazeutika und Pflanzenschutzmitteln. Die Anforderungen an Qualität und Reinheit sind deshalb sehr hoch. Insbesondere besteht für 3-Aminopropanol, das zur Herstellung von Panthenol und Panthenol-Derivaten, welche als Bestandteil von Salben in der Kosmetik und für medizinische Anwendungen eingesetzt wird, ein hoher Anspruch an den Geruch. Das eingesetzte 3-Aminopropanol darf nur einen geringen Eigengeruch aufweisen, da die Salben in der Regel direkt auf die menschliche Haut aufgetragen werden und ein Eigengeruch von vielen Kunden nicht akzeptiert werden würde.

Es wurde nun gefunden, dass 3-Aminopropanol, das durch konventionelle Verfahren, wie Destillation oder Rektifikation aufgereinigt wurde, den strengen Qualitätsanforderungen vieler Kunden der kosmetischen und pharmazeutischen Industrie nicht gerecht wird, da es einen zu starken Eigengeruch aufweist.

Die Aufgabe der vorliegenden Erfindung bestand in der Bereitstellung eines Verfahrens zur Aufreinigung von 3-Aminopropanol, wobei gegenüber dem Stand der Technik ein hochreines 3-Aminopropanol mit geringem Eigengeruch erhalten wird, das die Qualitätsnormen der kosmetischen und pharmazeutischen Industrie erfüllt.

Erfindungsgemäß wurde die Aufgabe gelöst durch ein Verfahren zur Aufreinigung eines 3-Aminopropanol enthaltenden Reaktionsaustrags, der bei der Umsetzung von Ethylencyanhydrin mit Wasserstoff in Gegenwart von Ammoniak anfällt, dadurch gekennzeichnet, dass man den 3-Aminopropanol enthaltenden Reaktionsaustrag in zwei oder mehr Stufen destilliert, wobei der Ammoniakgehalt des 3-Aminopropanol enthaltenden Reaktionsaustrags vor Einleitung in die erste Destillationsstufe 1 Gew.-% oder weniger beträgt und die Temperatur in den Destillationsstufen nicht mehr als 135°C beträgt, und man den Ammoniakgehalt des 3-Aminopropanol enthaltende Reaktionsaustrag vor der Zuführung in die erste Destillationsstufe durch Entgasung verringert, wobei die Entgasung in einer Rektifikationskolonne unter Einleitung von Stickstoff erfolgt.

Ein weiterer Gegenstand der vorliegenden Erfindung ist ein Verfahren zur Herstellung von 3-Aminopropanol durch Umsetzung von Ethylencyanhydrin mit Wasserstoff in Gegenwart von Ammoniak, dadurch gekennzeichnet, dass man die Aufreinigung des 3-Aminopropanol erfindungsgemäß durchführt.

3-Aminopropanol wird durch Umsetzung von Ethylencyanhydrin mit Wasserstoff in Gegenwart von Ammoniak erhalten.

Bevorzugt erfolgt die Umsetzung von 3-Aminopropanol mit Wasserstoff und Ammoniak in Gegenwart eines Katalysators.

In das erfindungsgemäße Verfahren wird Ethylencyanhydrin eingesetzt.

Die Herstellung von Ethylencyanhydrin kann über verschiedene Herstellungswege erfolgen. Bevorzugt wird in das erfindungsgemäße Verfahren Ethylencyanhydrin eingesetzt, das durch Umsetzung von Ethylenoxid mit Blausäure hergestellt wurde.

Ein solches Ethylencyanhydrin fällt beispielsweise als Zwischenprodukt bei der Herstellung von Acrylnitril an. Bei der Acrylnitrilherstellung wird im Allgemeinen Ethylenoxid mit Blausäure in basischer Umgebung zu Ethylencyanhydrin umgesetzt, welches unter Abspaltung eines Wassermoleküls an einem Al₂O₃-Katalysator zu Acrylnitril weiter umgesetzt werden kann.

Ethylencyanhydrin kann vor der Umsetzung mit Wasserstoff in Gegenwart von Ammoniak aufgereinigt werden, beispielsweise durch Destillation, es kann aber auch direkt - ohne weitere Aufarbeitung - in die Hydrierung eingesetzt werden.

In das erfindungsgemäße Verfahren wird Wasserstoff eingesetzt.

Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

In das erfindungsgemäße Verfahren wird weiterhin Ammoniak eingesetzt.

Als Ammoniak kann herkömmlich im Handel erhältliches Ammoniak eingesetzt werden, beispielsweise Ammoniak mit einem Gehalt von mehr 98 Gew.-% Ammoniak, bevorzugt mehr als 99 Gew.-% Ammoniak, bevorzugt mehr als 99,5 Gew.-%, insbesondere mehr als 99,9 Gew.-% Ammoniak.

Die Umsetzung von Ethylencyanhydrin mit Ammoniak und Wasserstoff erfolgt bevorzugt in Gegenwart eines Katalysators.

Als Katalysatoren zur Hydrierung der Nitril-Funktion des Cyanhydrins zum Aminopropnanol können insbesondere Katalysatoren eingesetzt werden, die als aktive Komponente ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten.

Die oben genannten Katalysatoren können in üblicher Weise mit Promotoren, beispielsweise mit Chrom, Eisen, Kobalt, Mangan, Molybdän, Titan, Zinn, Metallen der Alkaligruppe, Metallen der Erdalkaligruppe und/oder Phosphor dotiert werden.

Als Katalysatoren können sogenannte Skelett-Katalysatoren (auch als Raney^{®}-Typ bezeichnet, nachfolgend auch: Raney-Katalysator) eingesetzt werden, die durch Auslaugen (Aktivierung) einer Legierung aus hydrieraktivem Metall und einer weiteren Komponente (bevorzugt Al) erhalten werden. Bevorzugt werden Raney-Nickel-Katalysatoren oder Raney-Cobalt-Katalysatoren eingesetzt.

Als Katalysatoren können aber auch Katalysatoren eingesetzt werden, die durch Reduktion von sogenannten oxidischen Katalysatorvorläufern erhalten wurden.

In einer bevorzugten Ausführungsform werden in das erfindungsgemäße Verfahren Katalysatoren eingesetzt, die durch Reduktion von sogenannten Katalysatorvorläufern hergestellt werden. Der Katalysatorvorläufer enthält eine aktive Masse, die eine oder mehrere katalytisch aktive Komponenten, ggf. Promotoren und optional ein Trägermaterial enthält.

Bei den katalytisch aktiven Komponenten handelt es sich um sauerstoffhaltige Verbindungen der oben genannten Metalle, beispielsweise um deren Metalloxide bzw. Hydroxide, wie CoO, NiO, CuO und/oder deren Mischoxide.

Im Rahmen dieser Anmeldung wird der Begriff katalytisch aktive Komponenten für oben genannte sauerstoffhaltige Metallverbindungen verwendet, soll aber nicht implizieren, dass diese sauerstoffhaltigen Verbindungen an sich bereits katalytisch aktiv sind. Die katalytisch aktiven Komponenten weisen in der Regel erst nach erfolgter Reduktion eine katalytische Aktivität in der erfindungsgemäßen Umsetzung auf.

Die Katalysatorvorläufer können nach bekannten Verfahren, z.B. durch Fällung, Auffällung oder Imprägnierung hergestellt werden.

In einer bevorzugten Ausführungsform werden Katalysatorvorläufer in das erfindungsgemäße Verfahren eingesetzt, die durch Tränkung (Imprägnierung) von Trägermaterialien hergestellt werden (getränkte Katalysatorvorläufer).

Die Trägermaterialien, die bei der Imprägnierung verwendet werden, können beispielsweise in Form von Pulvern oder Formkörpern, wie Strängen, Tabletten, Kugeln oder Ringen, eingesetzt werden. Für Wirbelbettreaktoren geeignetes Trägermaterial wird vorzugsweise durch Sprühtrocknung erhalten.

Als Trägermaterialien kommen beispielsweise Kohlenstoff, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der entsprechenden katalytisch aktiven Komponenten oder Dotierelemente in Betracht, wie Co-Nitrat oder Co-Chlorid. Im Anschluss wird das getränkte Trägermaterial in der Regel getrocknet und ggf. calciniert.

Die Calcinierung wird im Allgemeinen bei Temperaturen zwischen 300 und 800°C, vorzugsweise 350 bis 600°C, insbesondere bei 450 bis 550°C ausgeführt

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.

Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

In einer weiteren bevorzugten Ausführungsform werden Katalysatorvorläufer über eine gemeinsame Fällung (Mischfällung) aller ihrer Komponenten hergestellt. Dazu wird in der Regel eine lösliche Verbindung der entsprechenden aktiven Komponente, der Dotierelemente und ggf. eine lösliche Verbindung eines Trägermaterials in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der aktiven Komponenten kommen üblicherweise die entsprechenden Metallsalze, wie die Nitrate, Sulfate, Acetate oder Chloride, der voranstehend genannten Metalle in Betracht.

Als lösliche Verbindungen eines Trägermaterials werden in der Regel wasserlösliche Verbindungen von Ti, Al, Zr, Si etc., beispielsweise die wasserlöslichen Nitrate, Sulfate, Acetate oder Chloride dieser Elemente, verwendet.

Als lösliche Verbindungen der Dotierelemente werden in der Regel wasserlösliche Verbindungen der Dotierelemente, beispielsweise die wasserlöslichen Nitrate, Sulfate, Acetate oder Chloride dieser Elemente, eingesetzt.

Katalysatorvorläufer können weiterhin durch Auffällung hergestellt werden.

Unter Auffällung wird eine Herstellmethode verstanden, bei der ein schwer lösliches oder unlösliches Trägermaterial in einer Flüssigkeit suspendiert wird und nachfolgend lösliche Verbindungen, wie lösliche Metallsalze, der entsprechenden Metalloxide zugegeben werden, welche dann durch Zugabe eines Fällungsmittels auf den suspendierten Träger aufgefällt werden (z.B. beschrieben in EP-A2-1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Kohlenstoffverbindungen, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Das Trägermaterial liegt in der Regel als Pulver oder Splitt vor.

Als Flüssigkeit, in der das Trägermaterial suspendiert wird, wird üblicherweise Wasser eingesetzt.

Als lösliche Verbindungen kommen die voranstehend genannten löslichen Verbindungen der aktiven Komponenten bzw. der Dotierelemente in Betracht.

Üblicherweise werden bei den Fällungsreaktionen die löslichen Verbindungen durch Zugabe eines Fällungsmittels als schwer- oder unlösliche, basische Salze gefällt.

Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100 °C, besonders 30 bis 90 °C, insbesondere bei 50 bis 70 °C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200°C, vorzugsweise 100 bis 150°C, getrocknet und anschließend calciniert.

Die Calcinierung wird im Allgemeinen bei Temperaturen zwischen 300 und 800°C, vorzugsweise 350 bis 600°C, insbesondere bei 450 bis 550°C ausgeführt.

Nach der Calcinierung werden die durch Fällungsreaktionen erhaltenen pulverförmigen Katalysatorvorläufer üblicherweise konditioniert.

Die Konditionierung kann beispielsweise dadurch erfolgen, dass man den Fällungskatalysator durch Vermahlen auf eine bestimmte Korngröße einstellt.

Nach der Vermahlung kann der durch Fällungsreaktionen erhaltenen Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise im Ullmann [Ullmann's Encyclopedia Electronic Release 2000, Kapitel: "Catalysis and Catalysts", Seiten 28-32] und von Ertl et al. [Ertl, Knözinger, Weitkamp, Handbook of Heterogenoeous Catalysis, VCH Weinheim, 1997, Seiten 98 ff] beschrieben.

Wie in den genannten Literaturstellen beschrieben, können durch den Prozess der Formgebung Formkörper in jeglicher Raumform, z.B. rund, eckig, länglich oder dergleichen, z.B. in Form von Strängen, Tabletten, Granulat, Kugeln, Zylindern oder Körnern erhalten werden. Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Nach der Konditionierung bzw. Formgebung erfolgt in der Regel eine Temperung. Die Temperaturen bei der Temperung entsprechen üblicherweise den Temperaturen bei der Calcinierung.

Die durch Fällungsreaktionen oder Imprägnierung erhaltenen Katalysatorvorläufer enthalten die katalytisch aktiven Komponenten in Form eines Gemisches ihrer sauerstoffhaltigen Verbindungen, d.h. insbesondere als Oxide, Mischoxide und/oder Hydroxide. Die so hergestellten Katalysatorvorläufer können als solche gelagert werden.

Besonders bevorzugt sind Katalysatorvorläufer, wie
die in EP-A-0636409 offenbarten Oxidgemische, die vor der Reduktion mit Wasserstoff 55 bis 98 Gew.-% Co, berechnet als CoO, 0,2 bis 15 Gew.-% Phosphor, berechnet als H₃PO₄, 0,2 bis 15 Gew.-% Mangan, berechnet als MnO₂, und 0,2 bis 5,0 Gew.-% Alkali, berechnet als M₂O (M=Alkali), enthalten, oder
in EP-A-0742045 offenbarte Oxidgemische, die vor der Reduktion mit Wasserstoff 55 bis 98 Gew.-% Co, berechnet als CoO, 0,2 bis 15 Gew.-% Phosphor, berechnet als H₃PO₄, 0,2 bis 15 Gew.-% Mangan, berechnet als MnO₂, und 0,05 bis 5 Gew.-% Alkali, berechnet als M₂O (M=Alkali), enthalten, oder in EP-A-696572 offenbarte Oxidgemische, die vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂, enthält, beispielsweise der in loc. cit, Seite 8, offenbarte Katalysator mit der Zusammensetzung 31,5 Gew.-% ZrO₂, 50 Gew.-% NiO, 17 Gew.-% CuO und 1,5 Gew.-% MoO₃, enthalten oder
in EP-A-963 975 offenbarte Oxidgemische, die vor der Reduktion mit Wasserstoff 22 bis 40 Gew.-% ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdäns enthält, beispielsweise der in loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO, enthalten, oder

Die Katalysatorvorläufer, die wie voranstehend beschrieben durch Imprägnierung oder Fällung hergestellt wurden, werden im Allgemeinen nach der Calcinierung bzw. Konditionierung reduziert. Durch die Reduktion wird der Katalysatorvorläufer in der Regel in seine katalytisch aktive Form umgewandelt.

Die Reduktion des Katalysatorvorläufers kann bei erhöhter Temperatur in einem bewegten oder unbewegten Reduktionsofen durchgeführt werden.

Als Reduktionsmittel wird üblicherweise Wasserstoff oder ein Wasserstoff enthaltendes Gas eingesetzt.

Der Wasserstoff kommt im Allgemeinen technisch rein zum Einsatz. Der Wasserstoff kann auch in Form eines Wasserstoff enthaltendem Gases, d.h. in Beimengungen mit anderen Inertgasen, wie Stickstoff, Helium, Neon, Argon oder Kohlendioxid zum Einsatz kommen. Der Wasserstoffstrom kann auch als Kreisgas in die Reduktion zurückgeführt werden, ggf. vermischt mit FrischWasserstoff und ggf. nach Entfernen von Wasser durch Kondensation.

Die Reduktion des Katalysatorvorläufers erfolgt bevorzugt in einem Reaktor, in dem die Katalysatorformkörper als Festbett angeordnet sind. Besonders bevorzugt erfolgt die Reduktion des Katalysatorvorläufers in demselben Reaktor in dem die nachfolgende Umsetzung von Ethylencyanhydrin mit Ammoniak erfolgt.

Weiterhin kann die Reduktion des Katalysatorvorläufers in einem Wirbelschichtreaktor in der Wirbelschicht erfolgen.

Die Reduktion des Katalysatorvorläufers erfolgt in der Regel bei Reduktionstemperaturen von 50 bis 600°C, insbesondere von 100 bis 500°C, besonders bevorzugt von 150 bis 450°C.

Der Wasserstoffpartialdruck beträgt in der Regel von 1 bis 300 bar, insbesondere von 1 bis 200 bar, besonders bevorzugt von 1 bis 100 bar, wobei sich die Druckangaben hier und im Folgenden auf den absolut gemessenen Druck beziehen.

Die Dauer der Reduktion beträgt bevorzugt 1 bis 20 Stunden, und besonders bevorzugt 5 bis 15 Stunden.

Während der Reduktion kann ein Lösungsmittel zugeführt werden, um entstehendes Reaktionswasser abzuführen und/oder um beispielsweise den Reaktor schneller aufheizen zu können und/oder während der Reduktion die Wärme besser abführen zu können. Das Lösungsmittel kann hierbei auch überkritisch zugeführt werden.

Geeignete Lösungsmittel können die zuvor beschriebenen Lösungsmittel eingesetzt werden. Bevorzugte Lösungsmittel sind Wasser; Ether wie Methyltertbutylether, Ethyltertbutylether, Dioxan oder Tetrahydrofuran. Besonders bevorzugt sind Wasser oder Tetrahydrofuran. Als geeignete Lösungsmittel kommen ebenfalls geeignete Mischungen in Betracht.

Die Reduktion des Katalysatorvorläufers kann auch in Suspension erfolgen, beispielsweise in einem Rührautoklaven. Die Temperaturen liegen im Allgemeinen in einem Bereich von 50 bis 300°C, insbesondere von 100 bis 250°C, besonders bevorzugt von 120 bis 200°C.

Die Reduktion in Suspension wird in der Regel bei einem Wasserstoffpartialdruck von 1 bis 300 bar, bevorzugt von 10 bis 250 bar, besonders bevorzugt von 30 bis 200 bar durchgeführt. Als Lösungsmittel kommen die voranstehend genannten Lösungsmittel in Betracht.

Die Dauer der Reduktion in Suspension beträgt bevorzugt 5 bis 20 Stunden, besonders bevorzugt 8 bis 15 Stunden.

Der so erhaltene Katalysator kann nach der Reduktion unter inerten Bedingungen gehandhabt werden. Bevorzugt kann der Katalysator unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Katalysator eingesetzt wird. Gegebenenfalls muss der Katalysator vor Beginn der eigentlichen Reaktion dann von der inerten Flüssigkeit befreit werden.

Die Lagerung des Katalysators unter inerten Substanzen ermöglicht eine unkomplizierte und ungefährliche Handhabung und Lagerung des Katalysators.

Der Katalysator kann nach der Reduktion aber auch mit einem Sauerstoff enthaltenden Gasstrom wie Luft oder einem Gemisch von Luft mit Stickstoff in Kontakt gebracht werden.

Dadurch wird ein passivierter Katalysator erhalten. Der passivierte Katalysator weist im Allgemeinen eine schützende Oxidschicht auf. Durch diese schützende Oxidschicht wird die Handhabung und Lagerung des Katalysators vereinfacht, so dass beispielsweise der Einbau des passivierten Katalysators in den Reaktor vereinfacht wird. Ein passivierter Katalysator wird bevorzugt vor dem Inkontakt bringen mit den Edukten wie oben beschrieben durch Behandlung des passivierten Katalysators mit Wasserstoff oder einem Wasserstoff enthaltenden Gas reduziert. Die Reduktionsbedingungen entsprechen im Allgemeinen den Reduktionsbedingungen, die bei der Reduktion der Katalysatorvorläufer angewandt werden. Durch die Aktivierung wird in der Regel die schützende Passivierungsschicht aufgehoben.

3-Aminopropanol wird durch Umsetzung von Ethylencyanhydrin mit Wasserstoff in Gegenwart von Ammoniak erhalten, wobei die Umsetzung bevorzugt in Gegenwart einer der oben genannten Katalysatoren erfolgt.

Das molare Verhältnis von eingesetztem Ammoniak zu eingesetztem Ethylencyanhydrin liegt üblicherweise in einem Bereich von 1:50 bis 100:1, bevorzugt, 1:1 bis 50:1, besonders bevorzugt 1,1:1 1 bis 25:1 und ganz besonders bevorzugt 2:1 bis 10:1.

Die Reaktion wird in der Regel bei einem Druck von 1 bis 500 bar, bevorzugt von 10 bis 400 bar, besonders von 100 bis 300 bar und ganz besonders bevorzugt von 120 bis 250 bar durchgeführt. Die Druckhaltung bzw. Drucksteuerung erfolgt in der Regel über die Dosierung des Wasserstoffs.

Die Hydrierung von Ethylencyanhydrin zu 3-Aminopropanol erfolgt im Allgemeinen bei Temperaturen von 20 bis 400°C, bevorzugt 20 bis 250°C, besonders bevorzugt 25 bis 200°C und ganz besonders bevorzugt 50 bis 150°C.

Die Umsetzung von Ethylencyanhydrin mit Ammoniak kann in Substanz oder in Anwesenheit eines Lösungsmittels erfolgen, z.B. in Ether, wie Methyl-tert-butylether, Ethyltertbutylether oder Tetrahydrofuran (THF); Alkoholen, wie Methanol, Ethanol oder Isopropanol; Kohlenwasserstoffen, wie Hexan, Heptan oder Raffinatschnitte; Aromaten, wie Toluol; Amide, wie Dimethylformamid oder Dimethylacetamid oder Lactamen, wie N-Methylpyrrolidon, N-Ethylpryrrolidon, N-Methylcaprolactam oder N-Ethylcaprolactam. Als Lösungsmittel kommen auch geeignete Mischungen der zuvor aufgeführten Lösungsmittel in Betracht. Das Lösungsmittel kann in einem Anteil von 5 bis 95 % Gew.-%, bevorzugt 20 bis 70 %, besonders bevorzugt 30 bis 60 %, eingesetzt werden, jeweils bezogen auf das Gesamtgewicht des Reaktionsgemisches, wobei das Gesamtgewicht des Reaktionsgemisches aus der Summe der Massen der in das Verfahren eingesetzten Ausgangsstoffe und Lösungsmittel ist.

Bevorzugt setzt man das Wertprodukt 3-Aminopropanol als Lösungsmittel ein, weil dies die Abtrennung des Lösemittels während der Aufarbeitung erspart.

In einer besonders bevorzugten Ausführungsform wird die Umsetzung von Ethylencyanhydrin mit Ammoniak in Substanz, d.h. ohne einen Zusatz von Lösungsmittel durchgeführt.

Das erfindungsgemäße Verfahren kann kontinuierlich, diskontinuierlich oder semi-kontinuierlich durchgeführt werden.

Bevorzugt wird das erfindungsgemäße Verfahren in einem Hochdruck-Rührkessel-Reaktor, Festbettreaktor oder Wirbelschichtreaktor durchgeführt.

In einer besonders bevorzugten Ausführungsform wird das erfindungsgemäße Verfahren in ein oder mehreren Festbettreaktoren durchgeführt.

Der Festbettreaktor kann sowohl in Sumpf- als in Rieselfahrweise betrieben werden. Bei der bevorzugten Rieselfahrweise wird bevorzugt ein Flüssigkeitsverteiler für den Reaktorfeed am Eingang des Reaktors eingesetzt. Wenn zwei Reaktoren eingesetzt werden, können beide in Sumpf- oder Rieselfahrweise betrieben werden. Alternativ kann der erste Reaktor in Sumpf-und der zweite Reaktor in Rieselfahrweise betrieben werden, oder umgekehrt.

Ethylencyanhydrin und Ammoniak können gemeinsam in die Reaktionszone des Reaktors gegeben werden, zum Beispiel als vorgemischter Reaktandenstrom. Die Zugabe kann auch getrennt voneinander erfolgen, wobei bei einem kontinuierlichen Verfahren die Edukte am Eingang des Reaktors eine Vermischung erfolgt, beispielsweise durch Flüssigkeitsverteiler oder entsprechende Einbauten. Bei einer diskontinuierlichen Ausführung können Ethylencyanhydrin und Ammoniak entweder gleichzeitig, zeitversetzt oder nacheinander in die Reaktionszone des Reaktors gegeben werden.

Die Verweilzeit beträgt bei der diskontinuierlich betriebenen Hydrierung von Ethylencyanhydrin im Allgemeinen 15 Minuten bis 24 Stunden, bevorzugt 30 Minuten bis 12 Stunden, besonders bevorzugt 30 Minuten bis 6 Stunden.

Bei Durchführung in einem bevorzugten kontinuierlichen Verfahren beträgt die Verweilzeit im Allgemeinen 0,1 Sekunden bis 24 Stunden, bevorzugt 1 Minute bis 10 Stunden, besonders bevorzugt 15 Minuten bis 300 Minuten und ganz besonders bevorzugt 15 Minuten bis 60 Minuten. Für die bevorzugten kontinuierlichen Verfahren bedeutet "Verweilzeit" in diesem Zusammenhang die Verweilzeit am Katalysator, für Festbettkatalysator somit die Verweilzeit im Katalysatorbett, für Wirbelschichtreaktoren wird der Syntheseteil des Reaktors (Teil des Reaktors, wo der Katalysator lokalisiert ist) betrachtet.

Vorzugsweise stellt man bei der kontinuierlichen Umsetzung von Ethylencyanhydrin mit Ammoniak eine Katalysatorbelastung von 0,01 bis 10, vorzugsweise von 0,05 bis 7, besonders bevorzugt von 0,1 bis 5 kg Ethylencyanhydrin pro kg Katalysator und Stunde ein.

Der 3-Aminopropanol enthaltende Reaktionsaustrag, der bei der Umsetzung von Ethylencyanhydrin mit Wasserstoff in Gegenwart von Ammoniak anfällt, enthält neben 3-Aminopropanol, nicht umgesetztes Ethylencyanhydrin, Wasser, geringe Mengen an Nebenprodukten sowie nicht umgesetztes Ammoniak.

Der Ammoniak-Gehalt des Reaktionsaustrags aus dem Hydrierreaktor liegt je nach eingesetzter Ammoniak-Menge im Bereich von 1 und 90 Gew.-%, bevorzugt 5 bis 80 Gew.-%, besonders bevorzugt 20 bis 70 Gew.-% und ganz besonders bevorzugt 40 bis 70 Gew.-%, jeweils bezogen auf die Masse des Reaktionsaustrages.

Der Austrag aus dem Hydrierreaktor wird erfindungsgemäß aufgearbeitet, in dem man den Reaktionsaustrag in zwei oder mehreren Stufen destilliert.

Im Rahmen der vorliegenden Erfindung hat sich herausgestellt, dass ein 3-Aminopropanol, das den strengen Qualitätsanforderungen der kosmetischen und pharmazeutischen Industrie genügt, nur dann erhältlich ist, wenn der Gehalt an Ammoniak des Reaktionsaustrages vor Einleitung in die erste Destillationsstufe 1 Gew.-% oder weniger beträgt und die Sumpftemperatur in den beiden Destillationsstufen nicht mehr als 135°C beträgt.

Wenn der Ammoniak-Gehalt des Reaktionsaustrags aus dem Hydrierreaktor mehr als 1 Gew.-% Ammoniak, bezogen auf die Gesamtmasse des Reaktionsaustrags enthält, muss der Ammoniakgehalt des Reaktionsaustrags aus dem Hydrierreaktor vor der Einleitung in die erste Destillationsstufe auf 1 Gew.-% oder weniger verringert werden.

In einer bevorzugten Ausführungsform wird der Ammoniak-Gehalt des Austrags aus dem Hydrierreaktor verringert, in dem der Reaktionsaustrag aus dem Hydrierreaktor in eine Destillationskolonne eingeleitet wird (Ammoniak-Abtrennung).

Die Ammoniak-Abtrennung erfolgt bevorzugt in einer Druckkolonne, wobei der Kolonnendruck so gewählt wird, dass der Ammoniak mit dem vorhandenen Kühlmedium bei der gegebenen Kühlmediumstemperatur, z.B. Kühlwasser, kondensiert werden kann.

Die Ammoniak-Abtrennung erfolgt bevorzugt in einer Destillationskolonne, die Einbauten zur Erhöhung der Trennleistung aufweist.

Die Ammoniak-Abtrennung wird besonders bevorzugt in einer Bodenkolonne durchgeführt, da solche Kolonnen für den Betrieb bei hohem Druck gut geeignet sind.

Bei einer Bodenkolonne befinden sich im Inneren der Kolonne Zwischenböden, auf denen der Stoffaustausch stattfindet. Beispiele für unterschiedliche Bodentypen sind Siebböden, Tunnelböden, Dualflowböden, Glockenböden oder Ventilböden.

Die destillativen Einbauten können aber auch als geordnete Packung vorliegen, beispielsweise als Blechpackung, wie Mellapak 250 Y oder Montz Pak, Typ B1-250, oder als strukturierte Keramikpackung oder als ungeordnete Packung, z.B. aus Pallringen, IMTP-Ringen (Fa. Koch-Glitsch), Raschig-Superringen, etc. Geordnete oder ungeordnete Packungen können in einem oder, bevorzugt, in mehreren Betten angeordnet sein.

Die genauen Betriebsbedingungen der Destillationskolonne können entsprechend der Trennleistung der verwendeten Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.

Der Reaktionsaustrag aus dem Hydrierreaktor wird vorzugsweise in einem räumlichen Bereich zwischen 30% und 90% der theoretischen Böden der Destillationskolonne zugeführt (von unten gezählt), besonders bevorzugt in einem räumlichen Bereich zwischen 50% und 80% der theoretischen Böden der Destillationskolonne. Beispielsweise kann die Zuführung etwas oberhalb der Mitte der theoretischen Böden erfolgen. Die optimale Zulaufstelle kann vom Fachmann in Abhängigkeit der Ammoniak-Konzentration mit den üblichen Berechnungswerkzeugen ermittelt werden.

Die Anzahl der theoretischen Böden liegt im Allgemeinen im Bereich von 5 bis 30, vorzugsweise 10 bis 20.

Der Kopfdruck beträgt besonders bevorzugt 1 bis 30 bar, besonders bevorzugt 10 bis 25 bar und insbesondere bevorzugt 15 bis 20 bar.

Im Kolonnensumpf wird bevorzugt eine Temperatur eingestellt, die oberhalb der Verdampfungstemperatur des Ammoniaks liegt, so dass Ammoniak vollständig oder weitestgehend vollständig in die Gasphase übergeht.

Besonders bevorzugt wird eine Temperatur eingestellt, die nahezu der Siedetemperatur des über Sumpf abzutrennenden Gemisches bei Kolonnensumpfdruck entspricht. Die Temperatur hängt von der Art und Zusammensetzung der im Sumpfprodukt enthaltenen Stoffe ab und kann vom Fachmann mit den üblichen thermodynamischen Berechnungswerkzeugen ermittelt werden.

Bevorzugt wird eine Temperatur von 165 bis 200°C, besonders bevorzugt 175 bis 195°C und insbesondere bevorzugt 180 bis 190°C eingestellt. Beispielsweise kann bei einem Kolonnenkopfdruck von 17 bar, bevorzugt eine Kolonnensumpftemperatur von 185°C eingestellt werden.

Der Kondensator der Destillationskolonne wird in der Regel bei einer Temperatur betrieben, in dem der überwiegende Teil des Ammoniaks bei dem entsprechenden Kopfdruck kondensiert wird. In der Regel liegt die Betriebstemperatur des Kondensators im Bereich von 25 bis 70°C, vorzugsweise 25 bis 45°C.

Der Rücklauf am Kolonnenkopf wird in der Regel so eingestellt, dass die überwiegende Menge an 3-Aminopropanol sowie Wasser in der Kolonne zurückgehalten werden, so dass sie praktisch vollständig als Sumpfprodukt erhalten werden. Vorzugsweise wird das am Kondensator anfallende Kondensat zu weniger als 50 %, bevorzugt zu weniger als 25 % in den Kopf der Destillationskolonne zurückgeführt.

Die für die Verdampfung erforderliche Energie wird üblicherweise durch einen Verdampfer im Kolonnensumpf eingetragen.

Im Kondensator fällt als Kondensat überwiegend Ammoniak an.

Das als Kondensat anfallende Ammoniak kann nach einer Aufreinigung oder vorzugsweise direkt als Ausgangsstoff für weitere chemische Synthesen eingesetzt. Beispielsweise kann der als Kondensat anfallende Ammoniak zur Herstellung von 3-Aminopropanol wiederverwendet werden, in dem der Ammoniak den 3-Aminopropanol-Herstellungsprozess zurückgeführt wird.

Als Sumpfaustrag aus der Ammoniak-Abtrennung wird im Allgemeinen ein Gemisch erhalten, welches 3-Aminopropanol, Wasser und in der Regel höher siedende Amine sowie organische Nebenprodukte enthält.

Weiterhin enthält der Sumpfaustrag aus der Ammoniak-Abtrennung in der Regel weniger als 10 Gew.-% und bevorzugt weniger als 5 Gew.-% Rest-Ammoniak.

Der Ammoniak-Gehalt des Austrags aus der Ammoniak-Abtrennung wird durch Entgasung weiter reduziert (Ammoniak-Entgasung).

Zur Entgasung wird der 3-Aminopropanol enthaltende Reaktionsaustrag mit einem Stripgas behandelt und kann gegebenenfalls entspannt und/oder erwärmt werden.

Die Entgasung von Ammoniak erfolgt in einer Entgasungskolonne.

Die Entgasung erfolgt in einer hierfür üblichen Apparatur, wie sie beispielsweise in: Kirk-Othmer, Encyclopedia of Chemical Technology, 3. Ed., Vol. 7, John Wiley & Sons, New York, 1979, Seite 870-881 beschrieben ist, nämlich einer Rektifikationskolonne, beispielsweise Siebbodenkolonne, Glockenbodenkolonne, Packungskolonne oder Füllkörperkolonne.

Vorzugsweise erfolgt die Entgasung des 3-Aminopropanolenthaltenden Reaktionsaustrags der Ammoniak-Abtrennung in einer Destillationskolonne mit Abtriebs- und Verstärkungsteil, wobei der 3-Aminopropanol enthaltenden Reaktionsaustrag bevorzugt im oberen Bereich der Kolonne eingespeist wird und am Kolonnensumpf der Ammoniak-abgereicherte Reaktionsaustrag abgezogen wird, der dann erfindungsgemäß einer zwei- oder mehrstufigen Destillation zugeführt werden kann.

Am Kolonnenkopf wird in der Regel ein gasförmiger Strom abgezogen, der im Wesentlichen Ammoniak enthält.

Die genauen Betriebsbedingungen der Entgasungskolonne können entsprechend der Trennleistung der verwendeten Kolonne vom Fachmann anhand der bekannten Dampfdrucke und Verdampfungsgleichgewichte der im 3-Aminopropanol enthaltenden Reaktionsaustrag enthaltenen Komponenten nach herkömmlichen Berechnungsmethoden routinemäßig ermittelt werden.

Die Ammoniak-Entgasung erfolgt bevorzugt in einer Destillationskolonne, die Einbauten zur Erhöhung der Trennleistung aufweist.

Die Ammoniak-Entgasung wird besonders bevorzugt in einer Bodenkolonne durchgeführt. Bei einer Bodenkolonne befinden sich im Inneren der Kolonne Zwischenböden, auf denen der Stoffaustausch stattfindet. Beispiele für unterschiedliche Bodentypen sind Siebböden, Tunnelböden, Dualflowböden, Glockenböden oder Ventilböden.

In einer weiteren bevorzugten Ausführungsform können die destillativen Einbauten aber auch als geordnete Packung vorliegen, beispielsweise als Blechpackung, wie Mellapak 250 Y oder Montz Pak, Typ B1-250, oder als strukturierte Keramikpackung oder als ungeordnete Packung, z.B. aus Pallringen, IMTP-Ringen (Fa. Koch-Glitsch), Raschig-Superringen, etc. Geordnete oder ungeordnete Packungen können in einem oder, bevorzugt, in mehreren Betten angeordnet sein.

Das Rohprodukt aus der Ammoniak-Abtrennung wird vorzugsweise in einem räumlichen Bereich zwischen 50% und 90% der theoretischen Böden der Destillationskolonne zugeführt (von unten gezählt), besonders bevorzugt in einem räumlichen Bereich zwischen 60% und 85% der theoretischen Böden der Destillationskolonne. Beispielsweise kann die Zuführung oberhalb der Mitte der theoretischen Böden erfolgen. Die optimale Zulaufstelle kann vom Fachmann in Abhängigkeit der Ammoniak-Konzentration mit den üblichen Berechnungswerkzeugen ermittelt werden.

Die Anzahl der theoretischen Böden liegt im Allgemeinen im Bereich von 10 bis 100, vorzugsweise 15 bis 80, besonders bevorzugt 20 bis 70 und ganz besonders bevorzugt 25 bis 60.

Der Kopfdruck beträgt besonders bevorzugt 500 bis 3000 mbar, besonders bevorzugt 800 bis 2000 mbar und ganz besonders bevorzugt 1000 bis 1500 mbar.

Im Kolonnensumpf wird bevorzugt eine Temperatur eingestellt, die oberhalb der Verdampfungstemperatur des Ammoniaks liegt, so dass Ammoniak vollständig oder weitestgehend vollständig in die Gasphase übergeht.

Besonders bevorzugt wird eine Temperatur eingestellt, die 135°C, bevorzugt 130°C und besonders bevorzugt 125°C nicht überschreitet.

Beispielsweise kann bei einem Kolonnenkopfdruck von 1013 mbar, bevorzugt eine Kolonnensumpftemperatur von 135°C eingestellt werden.

Die für die Verdampfung erforderliche Energie wird üblicherweise durch einen Verdampfer im Kolonnensumpf eingetragen.

In einer bevorzugten Ausführungsform wird in die Kolonne ein Strippgas eingeleitet. Strippgase sind Gase, die sich unter den vorliegenden Reaktionsbedingungen inert verhalten und nicht mit den im Reaktionsgemisch vorhandenen Stoffen reagieren. Als Strippgase können Inertgase, wie Stickstoff oder Edelgase (Helium, Neon, Argon, Xenon) eingesetzt werden. Bevorzugt wird Stickstoff als Inertgas verwendet.

Das Strippgas wird vorzugsweise in den unteren Bereich der Destillationskolonne eingeleitet und somit im Gegenstrom zum Flüssigkeitsstrom geführt.

Die Einleitung kann in den Sumpf der Kolonne erfolgen, beispielsweise mittels eines Verteilerringes oder einer Düse, sie kann aber auch in den unteren Bereich der Destillationskolonne, bevorzugt in einen räumlichen Bereich bis zu 30%, bevorzugt bis zu 20% und besonders bevorzugt bis zu 10% der theoretischen Böden der Destillationskolonne (von unten gezählt). Das eingeleitete Strippgas wird in der Regel durch die in der Kolonne vorhandenen Einbauten mit der entgegenströmenden Flüssigkeit gut durchmischt.

Der Strom an zugeführtem Inertgas beträgt bevorzugt 0,001 bis 1, besonders bevorzugt 0,005 bis 0,1 und ganz besonders bevorzugt 0,01 bis 0,05 m³/h Inertgas pro kg/h Zulauf.

Im oberen Bereich der Kolonne wird Ammoniak in der Regel als gasförmiger Strom abgezogen.

Das anfallende Ammoniak kann nach einer Aufreinigung oder vorzugsweise direkt als Ausgangsstoff für weitere chemische Synthesen eingesetzt werden, beispielsweise kann das anfallende Ammoniak in den Herstellungsprozess zurückgeführt werden.

Als Sumpfaustrag aus der Ammoniak-Entgasung wird im Allgemeinen ein Gemisch erhalten, welches 3-Aminopropanol, Rest-Ammoniak, Wasser sowie ggf. höher siedende Nebenkomponenten enthält.

Im unteren Bereich der Entgasungskolonne fällt in der Regel ein Austrag an, der einen Ammoniak-Gehalt im Bereich von 0,001 bis 1 Gew.-%, vorzugsweise 0,005 bis 0,5 Gew.-%, besonders bevorzugt im Bereich von 0,01 bis 0,25 Gew.-% und insbesondere bevorzugt im Bereich von 0,015 bis 0,1 Gew.-% aufweist, bezogen auf die Gesamtmasse des 3-Aminopropanol-enthaltenden Stroms.

Das ammoniak-haltige Abgas aus dem Stripprozess wird in der Regel aufgearbeitet oder der Entsorgung zugeführt.

Der Austrag aus der Ammoniak-Engasung, also der Austrag aus der Entgasungskolonne, kann als Zulauf in die zwei-oder mehrstufige Destillation als Zulaufstrom eingeleitet werden.

Wenn der Ammoniak-Gehalt des 3-Aminopropanol-enthaltenden Austrags aus der Ammoniak-Entgasung mehr als 1 Gew.-%, bevorzugt mehr als 0,5 Gew.-%, besonders bevorzugt mehr als 0,25 Gew.-% und insbesondere bevorzugt mehr als 0,1 Gew.-% beträgt, so sollte der Austrag aus der Entgasungskolonnen jedoch einem weiteren Entgasungsschritt unterzogen werden, um den Ammoniakgehalt weiter zu reduzieren, bevor er in die erfindungsgemäße Aufarbeitung eingeleitet wird.

Der Zulaufstrom, der in die zwei- oder mehrstufige Destillation eingeleitet wird, enthält in der Regel 3-Aminopropanol, Rest-Ammoniak, Wasser sowie ggf. höher siedende Nebenkomponenten.

Erfindungsgemäß weist der Zulaufstrom, der in die erfindungsgemäße zwei- oder mehrstufige Destillation eingeleitet wird, einen Ammoniakgehalt von 1 Gew.-% oder weniger auf, bezogen auf die Gesamtmasse des Zulaufstroms. Vorzugsweise sollte der Ammoniak-Gehalt weniger als 0,5 Gew.-%, besonders bevorzugt weniger als 0,25 Gew.-% und ganz besonders bevorzugt weniger als 0,1 Gew.-% betragen.

In der Regel sollte der Ammoniak-Gehalt des Zulaufstroms im Bereich von 0,001 bis 1 Gew.-%, vorzugsweise 0,005 bis 0,5 Gew.-%, besonders bevorzugt im Bereich von 0,01 bis 0,25 Gew.-% und insbesondere bevorzugt im Bereich von 0,015 bis 0,1 Gew.-% liegen.

Wie voranstehend beschrieben, sollte der Ammoniak-Gehalt in einem Zulauf, der einen Ammoniak-Gehalt von mehr als 1 Gew.-%, bevorzugt mehr als 0,5 Gew.-%, besonders bevorzugt mehr als 0,25 Gew.-% und insbesondere bevorzugt mehr als 0,1 Gew.-% aufweist, verringert werden, beispielsweise durch die zuvor beschriebene Ammoniak-Entgasung und/oder der Ammoniak-Abtrennung.

Vorzugsweise erfolgt die erste Stufe der Destillation (Wasserabtrennung) in einer Destillationskolonne, beispielsweise einer Siebbodenkolonne, Glockenbodenkolonne, Packungskolonne oder Füllkörperkolonne.

Besonders bevorzugt erfolgt die Destillation des Roh-Aminopropanols in einer Rektifikationskolonne mit Abtriebs- und Verstärkungsteil, wobei das Roh-Aminopropanol bevorzugt im Bereich der Kolonnenmitte eingespeist wird und am Kolonnensumpf ein Hochsiedergemisch abgezogen wird, welches überwiegend Aminopropanol und ggf. höhersiedende Nebenprodukte enthält. Am Kolonnenkopf wird ein flüssiger oder gasförmiger Strom abgezogen, der im Wesentlichen Wasser und Reste von Ammoniak enthält.

Die genauen Betriebsbedingungen können entsprechend der Trennleistung der verwendeten Kolonne vom Fachmann anhand der bekannten Dampfdrücke und Verdampfungsgleichgewichte der im Roh-Aminopropanol enthaltenen Komponenten nach herkömmlichen Berechnungsmethoden routinemäßig ermittelt werden.

Bevorzugt weist die Destillationskolonne Einbauten zur Erhöhung der Trennleistung auf. Die destillativen Einbauten können vorzugsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252.Y verwendet werden. Vorteilhaft bei der Verwendung dieser destillativen Einbauten sind der geringe Druckverlust und der geringe spezifische Flüssig-Hold-up im Vergleich zu beispielsweise Ventilböden. Die Einbauten können in ein oder mehren Betten vorliegen.

Der Zulaufstrom, der 3-Aminopropanol enthält, wird vorzugsweise in einem räumlichen Bereich zwischen 25% und 75%der theoretischen Böden der Destillationskolonne zugeführt (von unten gezählt), besonders bevorzugt in einem räumlichen Bereich zwischen 30% und 65% der theoretischen Böden der Destillationskolonne. Beispielsweise kann die Zuführung etwas unterhalb der Mitte der theoretischen Böden erfolgen. Die optimale Zulaufstelle kann vom Fachmann mit den üblichen Berechnungswerkzeugen ermittelt werden.

Die Anzahl der theoretischen Böden liegt im Allgemeinen im Bereich von 5 bis 50, vorzugsweise 20 bis 40.

Der Kopfdruck beträgt bevorzugt 5 bis 1000 mbar, besonders bevorzugt 10 bis 500 mbar, insbesondere bevorzugt 15 bis 100 mbar.

Im Kolonnensumpf wird bevorzugt eine Temperatur eingestellt, die oberhalb der Verdampfungstemperatur von Wasser liegt, aber unterhalb der Verdampfungstemperatur von 3-Aminopropanol.

Erfindungsgemäß beträgt die Temperatur im Kolonnensumpf jedoch nicht mehr als 135°C. Vorzugsweise beträgt die Temperatur im Kolonnensumpf 50 bis 130°C, besonders bevorzugt von 80 bis 125°C und insbesondere bevorzugt 100 bis 125°C.

Beispielsweise kann bei einem Kolonnenkopfdruck von 0,1 bar, bevorzugt eine Kolonnensumpftemperatur von 130°C eingestellt werden.

Der Kondensator der Destillationskolonne wird in der Regel bei einer Temperatur betrieben, in dem der überwiegende Teil des Wassers bei dem entsprechenden Kopfdruck kondensiert wird. In der Regel liegt die Betriebstemperatur des Kondensators im Bereich von 25 bis 70°C, vorzugsweise 30 bis 50°C.

Vorzugsweise wird das am Kondensator anfallende Kondensat zu mehr als 30 %, bevorzugt zu mehr als 40%, in den Kopf der Destillationskolonne zurückgeführt.

Die für die Verdampfung erforderliche Energie wird üblicherweise durch einen Verdampfer im Kolonnensumpf eingetragen.

Im Kondensator fällt ein Kondensat an, welches überwiegend Wasser und Rest-Ammoniak enthält.

Im Sumpfaustrag wird in der Regel ein Gemisch erhalten, das 3-Aminopropanol sowie ggf. höhere Nebenprodukte, enthält.

Der Sumpfaustrag aus der ersten Destillationsstufe wird im Rahmen der vorliegenden Erfindung als "Austrag der ersten Destillationsstufe" bezeichnet.

Der Austrag der ersten Destillationsstufe (Wasserabtrennung) wird erfindungsgemäß mindestens einer weiteren Destillationsstufe (Reindestillation) zugeführt.

Vorzugsweise erfolgt die zweite Stufe der Destillation ebenfalls in einer Destillationskolonne, beispielsweise Siebbodenkolonne, Glockenbodenkolonne, Packungskolonne oder Füllkörperkolonne.

Besonders bevorzugt erfolgt die Destillation des Austrags der ersten Destillationsstufe in einer Rektifikationskolonne mit Abtriebs- und Verstärkungsteil, wobei der Austrag der ersten Destillationsstufe bevorzugt im Bereich der Kolonnenmitte eingespeist wird und am Kolonnensumpf ein Hochsiedergemisch, welches überwiegend nicht umgesetztes Ethylencyanhydrin enthält, abgezogen wird. Am Kolonnenkopf wird ein flüssiger oder gasförmiger Strom abgezogen, der im Wesentlichen reines Aminopropanol enthält.

Die genauen Betriebsbedingungen können entsprechend der Trennleistung der verwendeten Kolonne vom Fachmann anhand der bekannten Dampfdrucke und Verdampfungsgleichgewichte der im Austrag der ersten Destillationsstufe enthaltenen Komponenten nach herkömmlichen Berechnungsmethoden routinemäßig ermittelt werden.

Bevorzugt weist die Destillationskolonne Einbauten zur Erhöhung der Trennleistung auf. Die destillativen Einbauten können vorzugsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252.Y verwendet werden. Vorteilhaft bei der Verwendung dieser destillativen Einbauten sind der geringe Druckverlust und der geringe spezifische Flüssig-Hold-up im Vergleich zu beispielsweise Ventilböden. Die Einbauten können in ein oder mehren Betten vorliegen.

Der Austrag der ersten Destillationsstufe , welcher 3-Aminopropanol sowie ggf. höher siedende Nebenkomponenten enthält, wird vorzugsweise in einem räumlichen Bereich zwischen 25% und 75%der theoretischen Böden der Destillationskolonne zugeführt (von unten gezählt), besonders bevorzugt in einem räumlichen Bereich zwischen 30% und 65% der theoretischen Böden der Destillationskolonne. Beispielsweise kann die Zuführung etwas unterhalb der Mitte der theoretischen Böden erfolgen. Die optimale Zulaufstelle kann vom Fachmann mit den üblichen Berechnungswerkzeugen ermittelt werden.

Die Anzahl der theoretischen Böden liegt im Allgemeinen im Bereich von 5 bis 100, vorzugsweise 30 bis 80.

Der Kopfdruck beträgt bevorzugt 5 bis 1000 mbar, besonders bevorzugt 10 bis 500 mbar, insbesondere bevorzugt 15 bis 100 mbar:
Im Kolonnensumpf wird bevorzugt eine Temperatur eingestellt, die oberhalb der Verdampfungstemperatur von 3-Aminopropanol liegt.

Erfindungsgemäß beträgt die Temperatur im Kolonnensumpf jedoch nicht mehr als 135°C. Vorzugsweise beträgt die Temperatur im Kolonnensumpf 50 bis 130°C, besonders bevorzugt von 80 bis 125°C und insbesondere bevorzugt 100 bis 125°C.

Beispielsweise kann bei einem Kolonnenkopfdruck von 40 mbar, bevorzugt eine Kolonnensumpftemperatur von 120°C eingestellt werden.

Beispielsweise kann bei einem Kolonnenkopfdruck von 70 mbar, bevorzugt eine Kolonnensumpftemperatur von 125°C.

Der Kondensator der Destillationskolonne wird in der Regel bei einer Temperatur betrieben, in dem der überwiegende Teil des 3-Aminopropanols bei dem entsprechenden Kopfdruck kondensiert wird. In der Regel liegt die Betriebstemperatur des Kondensators im Bereich von 25 bis 70°C, vorzugsweise 30 bis 50°C.

Vorzugsweise wird das am Kondensator anfallende Kondensat zu mehr als 80 %, bevorzugt zu mehr als 90%, in den Kopf der Destillationskolonne zurückgeführt.

Die für die Verdampfung erforderliche Energie wird üblicherweise durch einen Verdampfer im Kolonnensumpf eingetragen.

Im Sumpfaustrag wird in der Regel ein Gemisch erhalten, das die höher siedende Nebenkomponenten enthält.

Das als Kondensat der zweiten Destillationsstufe erhaltene 3-Aminopropanol muss in der Regel keiner weiteren Destillationsstufe unterzogen werden, kann jedoch nach Bedarf in einer oder mehreren weiteren Stufen destillativ aufgearbeitet werden.

Bevorzugt wird das als Kopfaustrag der zweiten Destillationsstufe erhaltene 3-Aminopropanol jedoch nicht weiter aufgearbeitet.

Das als Kopfaustrag der zweiten Destillationsstufe erhaltene 3-Aminopropanol weist bevorzugt eine Reinheit von mehr als 99 Gew.-%, besonders bevorzugt mehr als 99,5 Gew.-%, besonders bevorzugt mehr als 99,7 Gew.-% und insbesondere bevorzugt mehr als 99,9 Gew.-% auf.

Das erfindungsgemäß erhältliche 3-Aminopropanol kann für die Herstellung von 3-Aminopropanolderivaten verwendet werden. Insbesondere ist das erfindungsgemäß erhältliche 3-Aminopropanol zur Herstellung von Produkten für kosmetische und/oder therapeutische Anwendungen geeignet, speziell Panthenol, Acambrosate, Mefenorex, Domperidon, lfosamid oder Urapidil.

Der Wirkstoff Panthenol wird von vielen Herstellern als Inhaltsstoff für Hautcremes und Salben oder auch für Lutschtabletten, Nasensprays, Augentropfen und Kontaktlinsen-Reinigungsprodukte verwendet.

Demgemäß ist ein Gegenstand der vorliegenden Erfindung auch ein Verfahren zur Herstellung von 3-Aminopropanolderivaten, insbesondere Panthenol, Acambrosate, Mefenorex, Domperidon, lfosamid und Urapidil, wobei bei der Herstellung ein 3-Aminopropanol eingesetzt wird, dass in einem erfindungsgemäßen Verfahren hergestellt wird.

Die nach dem erfindungsgemäßen Verfahren erhaltene 3-Aminopropanol weist eine höhere Reinheit auf als ein nach bekannten Destillationsverfahren erhaltenes 3-Aminopropanol.

Insbesondere weist das erfindungsgemäße 3-Aminopropanol nur einen geringen Eigengeruch auf, so dass es als Ausgangsprodukt für die Herstellung von Salben, die in der Regel direkt auf die menschliche Haut aufgetragen werden, geeignet ist.

Das erfindungsgemäß erhältliche 3-Aminopropanol erfüllt die strengen und hohen Qualitätsnormen der kosmetischen und pharmazeutischen Industrie.

Die Erfindung wird durch die nachstehenden Beispiele näher erläutert.

### Beispiele:

### Allgemeine Vorschriften:

### Beispiel 1: Herstellung von 3-Aminopropanol

Ethylencyanhydrin (450Kg/h) wurden zusammen mit Ammoniak (850Kg/h) in Gegenwart von Wasserstoff bei einem Druck von 180 bar und einer Temperatur von 100°C in einem Rohrreaktor umgesetzt. Als Katalysator wurde ein Katalysator gemäß Beispiel A der EP-A-0742045 eingesetzt. Die Katalysatorbelastung betrug 0,3kg ECH D / kg Katalysator / Stunde.

Der Reaktionsaustrag wurde in eine Destillationskolonne eingeleitet, die bei 17 bar Kolonnenkopfdruck betrieben wurde (Ammoniak-Abtrennung). Die Destillationskolonne wies 12 theoretische Böden auf. Die Einspeisungsstelle lag im Bereich des 10. Boden. Die Sumpftemperatur betrug 185°C.

Der Austrag aus der Ammoniak-Abtrennung wurde gaschromatographisch analysiert und enthielt:
93 FL.-% 3-Aminopropnaol;
2,5 FL.-% Ammoniak;
3,0 FI.-% Dihydroxipropylamin
0,3 FI.-% Diaminopropylether
0,2 FI.-% Ethandiol

### Beispiel 2: Ammoniak-Entgasung

Der Reaktionsaustrag aus Beispiel 1 wurde in eine Entgasungskolonne eingeleitet. Die Entgasungskolonne wies 50 theoretische Böden auf. Die Einspeisungsstelle lag im Bereich des 25. Bodens. Der Zulauf betrug 1600Kg/h. Die Destillation wurde bei einem Druck von 1 bar abs. und einer Sumpftemperatur von 130°C betrieben. Als Strippgas wurde Stickstoff verwendet, welches über den unteren Bereich der Kolonnen eingespeist wurde. Der Strom an zugeführtem Stickstoff betrug 30 m³/h.

Der Austrag aus der Entgasungskolonne wurde gaschromatographisch analysiert und enthielt: 94,08 FL.-% 3-Aminopropanol
0,07 FL.-% Ammoniak
0,83 % Wasser

### Beispiel 3: Zweistufige Destillation

Der Austrag aus der Ammoniak-Entgasung (Beispiel 2) wurde in eine zweistufige Destillation eingeleitet. Die erste Destillationskolonne (Wasserabtrennung) wies 33 theoretische Böden auf. Die Einspeisungstelle lag im Bereich des 20. Bodens. Der Kolonnenkopfdruck betrug 70 mbar abs. Die Kolonnensumpftemperatur betrug 120°C. Am Kolonnenkopf wurden Wasser und Reste von Ammoniak kondensiert. Der Austrag am Kolonnensumpf enthielt 3-Aminopropanol und höher siedende Nebenprodukte (Roh-Aminopropanol).

Die Zusammensetzung des Sumpfaustrages wurde gaschromatographisch analysiert und betrug:
96,0 FL.-% 3-Aminopropnaol
0,8 FL.-% Ethandiol

Der Sumpfaustrag aus der Wasserabtrennung wurde in eine weitere Destillationskolonne geleitet (Reindestillation). Diese zweite Destillationskolonne (Reindestillation) wies 62 theoretische Böden auf. Die Einspeisungsstelle lag im Bereich des 40. Bodens. Der Kolonnenkopfdruck betrug 40 mbar. Die Kolonnensumpftemperatur betrug 122°C. Am Kolonnenkopf wurde reines 3-Aminopropanol destilliert. Der Austrag am Kolonnensumpf enthielt höher siedende Nebenprodukte.

Die Zusammensetzung des Kopfaustrages wurde gaschromatographisch analysiert und betrug: 99,95 FI.-% 3-Aminopropanol;

### Beispiel 4: Zweistufige Destillation

Der Austrag aus der 3-Aminopropanol-Herstellung (Beispiel 1) wurde direkt in eine zweistufige Destillation eingeleitet. Die erste Destillationskolonne (Wasserabtrennung) wies 33 theoretische Böden auf. Die Einspeisungstelle lag im Bereich des 20. Bodens. Der Kolonnenkopfdruck betrug 350 mbar abs.. Die Kolonnensumpftemperatur betrug 158°C. Am Kolonnenkopf wurden Wasser und Reste von Ammoniak kondensiert. Der Austrag am Kolonnensumpf enthielt 3-Aminopropanol und höher siedende Nebenprodukte (Roh-Aminopropanol).

Die Zusammensetzung des Sumpfaustrages wurde gaschromatographisch analysiert und betrug:
98,2 FI.-% 3-Aminopropanol;

Der Sumpfaustrag aus der Wasserabtrennung wurde in eine weitere Destillationskolonne geleitet (Reindestillation). Diese zweite Destillationskolonne (Reindestillation) wies 62 theoretische Böden auf. Die Einspeisungstelle lag im Bereich des 40. Bodens. Der Kolonnenkopfdruck betrug 180 mbar. Die Kolonnensumpftemperatur betrug 149°C. Am Kolonnenkopf wurde reines 3-Aminopropanol destilliert. Der Austrag am Kolonnensumpf enthielt höher siedende Nebenprodukte.

Die Zusammensetzung des Kopfaustrages wurde gaschromatographisch analysiert und betrug: 99,8 FI.-% 3-Aminopropanol;

### Beispiel 5: Ammoniak-Entgasung

Der Reaktionsaustrag aus Beispiel 1 wurde in eine Entgasungskolonne eingeleitet. Die Entgasungskolonne wies 33 theoretische Böden auf. Die Einspeisungsstelle lag im Bereich des 20. Bodens. Der Zulauf betrug 3000Kg/h. Die Destillation wurde bei einem Druck von 85 mbar abs. und einer Sumpftemperatur von 127°C betrieben. Als Strippgas wurde Stickstoff verwendet, welches über den unteren Bereich der Kolonnen eingespeist wurde. Der Strom an zugeführtem Stickstoff betrug 30 m³/h.

Der Austrag aus der Entgasungskolonne wurde gaschromatographisch analysiert und enthielt:
95,7 FL.-% 3-Aminopropanol
0,02 FL.-% Ammoniak

### Beispiel 6: Zweistufige Destillation

Der Austrag aus der Entgasungskolonne (Beispiel 2) wurde in eine zweistufige Destillation eingeleitet. Die erste Destillationskolonne (Wasserabtrennung) wies 33 theoretische Böden auf. Die Einspeisungsstelle lag im Bereich des 20. Bodens. Der Kolonnenkopfdruck betrug 70 mbar abs. Die Kolonnensumpftemperatur betrug 118°C. Am Kolonnenkopf wurden Wasser und Reste von Ammoniak kondensiert. Der Austrag am Kolonnensumpf enthielt 3-Aminopropanol und höher siedende Nebenprodukte (Roh-Aminopropanol).

Die Zusammensetzung des Sumpfaustrages wurde gaschromatographisch analysiert und betrug:
95,5 FL.-% 3-Aminopropnaol
0,8 FL.-% Ethandiol

Der Sumpfaustrag aus der Wasserabtrennung wurde in eine weitere Destillationskolonne geleitet (Reindestillation). Diese zweite Destillationskolonne (Reindestillation) wies 62 theoretische Böden auf. Die Einspeisungsstelle lag im Bereich des 40. Bodens. Der Kolonnenkopfdruck betrug 40 mbar. Die Kolonnensumpftemperatur betrug 120°C. Am Kolonnenkopf wurde reines 3-Aminopropanol destilliert. Der Austrag am Kolonnensumpf enthielt höher siedende Nebenprodukte.

Die Zusammensetzung des Kopfaustrages wurde gaschromatographisch analysiert und betrug: 99,95 FI.-% 3-Aminopropanol;

Die Austräge der Beispiele wurden einer olfaktorischen Beurteilung unterzogen.

Hierzu wurde das in den Beispielen 3, 4 und 6 erhaltene 3-Aminopropanol zu Panthenol umgesetzt und dieses dann geruchlich beurteilt. Bei der geruchlichen Beurteilung wurde folgendes Protokoll angewandt:

### Herstellung von Panthenol:

In einem 1 l Vierhalskolben werden 150 g 3-Aminopropanol vorgelegt. Unter Rühren wurde bei Raumtemperatur langsam 260 g D-Pantolacton zugegeben. Nach beendeter Zugabe wurde die Reaktionsmischung auf 60°C erwärmt und für 5 weitere Stunden gerührt. Das eingesetzte D-Pantolacton wurde vorher 2 mal mit Methyl-tert.-Butylether (MTBE) gewaschen und anschlie-βend getrocknet.

Das durch die Umsetzung von 3-Aminopropanol und D-Pantolacton erhaltene roh-Panthenol wurde anschließend entgast und destilliert.

Die Entgasung wurde in einem Dünnfilmverdampfer bei einem Druck von 0,027 mbar, einer Sumpftemperatur von 80°C und einer Lamellendrehzahl von 280 Upm durchgeführt. Nach der Entgasung wurde die Apparatur durch mehrmaliges Spülen mit VE-Wasser und 2-Propanol mit anschließender Trocknung im Vakuum gereinigt

Anschließend wurde das entgaste Panthenol destilliert. Die Destillation wurde in derselben Apparatur, in der bereits die Entgasung vorgenommen wurde, durchgeführt. Die Sumpftemperatur betrug 120°C, der Druck 0,027 mbar und die Lamellendrehzahl 800 Upm.

Am Innenkondensator wurde bei einer Kühlwendeltemperatur von 60°C Panthenol erhalten.

75 g des destillierten Panthenols wurden mit 24,1 g destilliertem Wasser bei 40°C homogenisiert. Die so hergestellte wässrige Panthenollösung wird nachfolgend als Prüfprobe bezeichnet.

Das so erhaltene Panthenol (Prüfprobe) wurde olfaktorisch beurteilt.

### Olfaktorische Beurteilung:

Die sensorische Prüfung wurde in einer einfach oder im Zweifelsfall in einer Wiederholungsbestimmung durch geschultes Personal anhand einer validierten Methode durchgeführt. Im Zuge der Validierung wurde an einem praxisnahen Modellfall in einer 9-fach-Wiederholung ein Signifikanzniveau = 0,05 (statistisches Auswerteverfahren nach dem Binominaltheorem) vorgegeben und bestätigt.

3 ml der Prüfprobe wurden in ein Deckelglas (Durchmesser 70 mm, Höhe 120 mm, Inhalt 370 ml) mit einer Einwegpipette aus Polyethylen (Typ Makro 155, graduiert bis 3.0 ml) pipettiert.

In einem weiteren Deckelglas wurde in gleicher Weise eine geruchlich akzeptierte Referenzprobe als sogenannter Standard zubereitet.

Die Deckelgläser wurden verschlossen und konditioniert (10 Minuten bei Raumtemperatur). Hierdurch wird gewährleistet, dass sich ein Gleichgewicht zwischen flüssiger Phase und der mit flüchtigen Bestandteilen angereicherten Gasphase einstellen kann. Die Konditionierungszeiten sind mit einer Toleranz von +/- 1 Minute einzuhalten.

Nach der abgeschlossenen Konditionierung öffnete der Prüfer das Deckelglas des Standards, nahm den Geruch des Gasraumes auf und verschloss das Glas wieder. Ohne Zeitverzögerung wurde nun das Glas der Prüfprobe geöffnet und in gleicher Weise abgerochen und hiernach auch wieder verschlossen.

Vor einer Wiederholungsmessung wurden die Proben wieder konditioniert werden.

Soweit der Prüfer keinen geruchlichen Unterschied zum Standard festgestellt hatte, wurde die Probe mit dem Prädikat "ok" oder "ja" beurteilt.

Wurden produktuntypische Abweichungen zum Standard festgestellt, die den vorgesehen Einsatzzweck in Frage stellen, wurde die Probe mit dem Prädikat "oos" oder "nein" bewertet.

**Tabelle 1: Ergebnis der olfaktorischen Beurteilung:**

| Beispiel | NH₃-Gehalt des Zulaufs, der der Aufarbeitung zugeführt wird. | Sumpftemp. bei NH₃-Entgasung | Sumpftemp. der ersten Dest.-Stufe (Wasserabtrennung) | Sumpftemp. der zweiten Dest.-Stufe (Reindestillation) | Olfaktorische Beurteilung |
|---|---|---|---|---|---|
| 3 | 0,02 | 130 | 120 | 122 | ok |
| 4 | 2,5 | - | 158 | 149 | oos |
| 6 | 0,02 | 127 | 118 | 120 | o.k |

## Patentansprüche

1. Verfahren zur Aufreinigung eines 3-Aminopropanol enthaltenden Reaktionsaustrags, der bei der Umsetzung von Ethylencyanhydrin mit Wasserstoff in Gegenwart von Ammoniak anfällt, **dadurch gekennzeichnet, dass** man den 3-Aminopropanol enthaltenden Reaktionsaustrag in zwei oder mehr Stufen destilliert, wobei der Ammoniakgehalt des 3-Aminopropanol enthaltenden Reaktionsaustrags vor Einleitung in die erste Destillationsstufe 1 Gew.-% oder weniger beträgt und die Temperatur in den Destillationsstufen nicht mehr als 135°C beträgt, und man den Ammoniakgehalt des 3-Aminopropanol enthaltende Reaktionsaustrag vor der Zuführung in die erste Destillationsstufe durch Entgasung verringert, wobei die Entgasung in einer Rektifikationskolonne unter Einleitung von Stickstoff erfolgt.

2. Verfahren nach Anspruch 1, **dadurch gekennzeichnet dass** der 3-Aminopropanol enthaltenden Zulaufstrom, der in die erste Destillationsstufe eingeleitet wird, einen Ammoniakgehalt von 0,1 Gew.-% oder weniger aufweist

3. Verfahren gemäß mindestens einem der Ansprüche 1 bis 2, **dadurch gekennzeichnet, dass** Ethylencyanhydrin durch Umsetzung von Ethylenoxid und Blausäure hergestellt wird.

4. Verfahren gemäß mindestens einem der Ansprüche 1 bis 3, **dadurch gekennzeichnet, dass** die Umsetzung von Ethylencyanhydrin mit Wasserstoff in Gegenwart von Ammoniak in Gegenwart eines Katalysators erfolgt, der durch Reduktion eines Katalysatorvorläufers erhalten wird.

5. Verfahren gemäß Anspruch 4, **dadurch gekennzeichnet, dass** der Katalysatorvorläufer CoO, NiO, CuO, RuO(OH)ₓ oder LiCoO₂ als katalytisch aktive Komponenten enthält.

6. Verfahren gemäß Anspruch 5, **dadurch gekennzeichnet, dass** die katalytisch aktive Masse des Katalysatorvorläufers vor der Reduktion mit Wasserstoff 55 bis 98 Gew.-% Co, berechnet als CoO, 0,2 bis 15 Gew.-% Phosphor, berechnet als H₃PO₄, 0,2 bis 15 Gew.-% Mangan, berechnet als MnO₂, und 0,2 bis 15 Gew.-% Alkali, berechnet als M₂O (M=Alkali), enthält.

7. Verfahren gemäß mindestens einem der Ansprüche 1 bis 6, **dadurch gekennzeichnet, dass** die Umsetzung von Ethylencyanhydrin mit Wasserstoff in Gegenwart von Ammoniak in einem Festbettreaktor erfolgt.

8. Verfahren gemäß mindestens einem der Ansprüche 1 bis 7, **dadurch gekennzeichnet, dass** das molare Verhältnis von eingesetztem Ammoniak zu eingesetztem Ethylencyanhydrin in einem Bereich von 1:1 bis 50:1 zu liegt..

9. Verfahren gemäß mindestens einem der Ansprüche 1 bis 8, **dadurch gekennzeichnet, dass** die Sumpftemperatur in der ersten und/oder zweiten Destillationsstufe 100 bis 125°C beträgt.

10. Verfahren zur Herstellung von 3-Aminopropanol durch Umsetzung von Ethylencyanhydrin mit Wasserstoff in Gegenwart von Ammoniak, **dadurch gekennzeichnet, dass** man die Aufreinigung des 3-Aminopropanol enthaltenden Reaktionsaustrags gemäß mindestens einem der Ansprüche 1 bis 9 durchführt.

11. Verfahren zur Herstellung von Produkten für kosmetische und/oder therapeutische Anwendungen, **dadurch gekennzeichnet, dass** die Herstellung des bei der Herstellung von Produkten für kosmetische und/oder therapeutische Anwendungen eingesetzten 3-Aminopropanols gemäß mindestens einem der Ansprüche 1 bis 10 erfolgt.

12. Verfahren zur Herstellung von Produkten für kosmetische und/oder therapeutische Anwendungen nach Anspruch 11, **dadurch gekennzeichnet, dass** die Produkte für kosmetische und/oder therapeutische Anwendungen Panthenol, Acambrosat, Mefenorex, Domperidon, Ifosamid oder Urapidil sind.

## Claims

1. A process for purifying a reaction output which comprises 3-aminopropanol and is obtained in the reaction of ethylene cyanohydrin with hydrogen in the presence of ammonia, which comprises distilling the reaction output comprising 3-aminopropanol in two or more stages, the ammonia content of the reaction output comprising 3-aminopropanol before introduction into the first distillation stage being 1% by weight or less and the temperature in the distillation stages being not more than 135°C, and reducing the ammonia content of the reaction output comprising 3-aminopropanol before it is fed into the first distillation stage by degassing, the degassing being effected in a rectification column with introduction of nitrogen.

2. The process according to claim 1, wherein the feedstream which comprises 3-aminopropanol and is introduced into the first distillation stage has an ammonia content of 0.1% by weight or less.

3. The process according to at least one of claims 1 and 2, wherein ethylene cyanohydrin is prepared by reaction of ethylene oxide and hydrogen cyanide.

4. The process according to at least one of claims 1 to 3, wherein ethylene cyanohydrin is reacted with hydrogen in the presence of ammonia in the presence of a catalyst which is obtained by reduction of a catalyst precursor.

5. The process according to claim 4, wherein the catalyst precursor comprises CoO, NiO, CuO, RuO(OH)ₓ or LiCoO₂ as catalytically active components.

6. The process according to claim 5, wherein the catalytically active mass of the catalyst precursor, before it is reduced with hydrogen, comprises 55 to 98% by weight of Co, calculated as CoO, 0.2 to 15% by weight of phosphorus, calculated as H₃PO₄, 0.2 to 15% by weight of manganese, calculated as MnO₂, and 0.2 to 15% by weight of alkali metal, calculated as M₂O (M= alkali metal).

7. The process according to at least one of claims 1 to 6, wherein ethylene cyanohydrin is reacted with hydrogen in the presence of ammonia in a fixed bed reactor.

8. The process according to at least one of claims 1 to 7, wherein the molar ratio of ammonia used to ethylene cyanohydrin used is within a range from 1:1 to 50:1.

9. The process according to at least one of claims 1 to 8, wherein the bottom temperature in the first and/or second distillation stage is 100 to 125°C.

10. A process for preparing 3-aminopropanol by reacting ethylene cyanohydrin with hydrogen in the presence of ammonia, which comprises performing the purification of the reaction output comprising 3-aminopropanol according to at least one of claims 1 to 9.

11. A process for preparing products for cosmetic and/or therapeutic uses, which comprises preparing the 3-aminopropanol used in the preparation of products for cosmetic and/or therapeutic uses according to at least one of claims 1 to 10.

12. A process for preparing products for cosmetic and/or therapeutic uses according to claim 11, wherein the products for cosmetic and/or therapeutic uses are panthenol, acambrosate, mefenorex, domperidon, ifosamid or urapidil.

## Revendications

1. Procédé pour la purification d'un produit de réaction contenant du 3-aminopropanol, qui est obtenu lors de la transformation d'éthylènecyanhydrine avec de l'hydrogène en présence d'ammoniaque, **caractérisé en ce qu'**on distille le produit de réaction contenant du 3-aminopropanol en deux étapes ou plus, la teneur en ammoniaque du produit de réaction contenant du 3-aminopropanol, avant l'introduction dans la première étape de distillation, étant de 1% en poids ou moins et la température dans les étapes de distillation n'étant pas supérieure à 135°C et la teneur en ammoniaque du produit de réaction contenant du 3-aminopropanol étant abaissée par dégazage avant l'alimentation dans la première étape de distillation, le dégazage ayant lieu dans une colonne de rectification avec introduction d'azote.

2. Procédé selon la revendication 1, **caractérisé en ce que** le flux d'alimentation, contenant du 3-aminopropanol, qui est introduit dans la première étape de distillation, présente une teneur en ammoniaque de 0,1% en poids ou moins.

3. Procédé selon au moins l'une quelconque des revendications 1 à 2, **caractérisé en ce qu'**on prépare de l'éthylènecyanhydrine par transformation d'oxyde d'éthylène et d'acide cyanhydrique.

4. Procédé selon au moins l'une quelconque des revendications 1 à 3, **caractérisé en ce que** la transformation d'éthylènecyanhydrine avec de l'hydrogène en présence d'ammoniaque a lieu en présence d'un catalyseur qui est obtenu par réduction d'un précurseur catalytique.

5. Procédé selon la revendication 4, **caractérisé en ce que** le précurseur catalytique contient CoO, NiO, CuO, RuO(OH)ₓ ou LiCoO₂ comme composants catalytiquement actifs.

6. Procédé selon la revendication 5, **caractérisé en ce que** la masse catalytiquement active du précurseur catalytique, avant la réduction avec de l'hydrogène, contient 55 à 98% en poids Co, calculés sous forme de CoO, 0,2 à 15% en poids de phosphore, calculés sous forme de H₃PO₄, 0,2 à 15% en poids de manganèse, calculés sous forme de MnO₂, et 0,2 à 15% en poids d'alcalis, calculés sous forme de M₂O (M=alcali).

7. Procédé selon au moins l'une quelconque des revendications 1 à 6, **caractérisé en ce que** la transformation d'éthylènecyanhydrine avec de l'hydrogène en présence d'ammoniaque a lieu dans un réacteur à lit fixe.

8. Procédé selon au moins l'une quelconque des revendications 1 à 7, **caractérisé en ce que** le rapport de l'ammoniaque utilisé à l'éthylènecyanhydrine utilisée se situe dans une plage de 1:1 à 50:1.

9. Procédé selon au moins l'une quelconque des revendications 1 à 8, **caractérisé en ce que** la température du fond dans la première étape et/ou la deuxième étape de distillation est de 100 à 125°C.

10. Procédé pour la préparation de 3-aminopropanol par transformation d'éthylènecyanhydrine avec de l'hydrogène en présence d'ammoniaque, **caractérisé en ce qu'**on réalise la purification du produit de réaction contenant du 3-aminopropanol selon au moins l'une quelconque des revendications 1 à 9.

11. Procédé pour la préparation de produits destinés à des applications cosmétiques et/ou thérapeutiques, **caractérisé en ce que** la préparation du 3-aminopropanol utilisé lors de la préparation de produits destinés à des applications cosmétiques et/ou thérapeutiques a lieu selon au moins l'une quelconque des revendications 1 à 10.

12. Procédé pour la préparation de produits pour des utilisations cosmétiques et/ou thérapeutiques selon la revendication 11, **caractérisé en ce que** les produits destinés à des applications cosmétiques et/ou thérapeutiques sont le panthénol, l'acambrosate, le méfénorex, le dompéridon, l'ifosamide ou l'urapidil.
